# EUROPEAN PATENT SPECIFICATION

(11) **EP 1 210 948 B1**
(45) Date of publication and mention of the grant of the patent: **25.04.2007**
(21) Application number: 00957052.4
(22) Date of filing: 08.09.2000
(51) Int. Cl.: A61K 38/08, A61P 25/28, A61P 25/00, A61P 21/02, A61P 21/04, A61P 43/00

(54) **THERAPEUTC PEPTIDE FOR NERVOUS DISEASES**
PEPTID ZUR BEHANDLUNG VON NERVENERKRANKUNGEN
PEPTIDE POUR LA THERAPIE DES MALADIES NERVEUSES

(30) Priority: 09.09.1999 JP 25515999
(43) Date of publication of application: 05.06.2002
(73) Proprietor: NIPPON CHEMIPHAR CO., LTD., Tokyo 101-8678 (JP)
(72) Inventor: NISHIKAWA, Takashige, Kagoshima-shi,Kagoshima 890-0073 (JP); HIRATE, Kenji, Kasukabe-shi, Saitama 344-0025 (JP)
(74) Representative: Albrecht, Thomas
(86) International application number: PCT/JP2000/006150
(87) International publication number: WO 2001/017545

(56) References cited:
- EP-A1- 0 393 934
- JP-A- 11 171 786

## Description

### [FIELD OF THE INVENTION]

The present invention relates to an agent for treating nerval diseases and an inhibitor of glutamate toxicity (not including an antidementia agent) which contains a peptide or a pharmacologically acceptable salt thereof as an effective component.

### [BACKGROUND OF THE INVENTION]

The physiologic functions of living body are easily influenced by excessive or inappropriate stimulation by an excitatory neurotransmitter which is represented by a glutamate. It is known that abnormal excitatory neuro-transmitting system deeply relates to various nerval diseases including acute nerval diseases such as paroxysm, brain ischemia, spinal damage, injury of head, antepartum or postpartum anoxia, brain defect after heart bypass operation or heart transplantation, cardiac standstill, and hypoglycemia. Further, it is reported that the abnormal excitatory neuro-transmitting system pertains to chronic nerval diseases such as Alzheimer's disease, Huntington's chorea, amyotrophy, scleroma of lateral funiculus of spinal, dementia following AIDS, injury of eyes, retinopathy, agnosia, and Parkinson's disease. It is further reported that the abnormal excitatory neuro-transmitting system pertains to muscle spasm, convulsion, migraine, incontinence of urine, breakaway from nicotine, mental diseases such as schizophrenia, epilepsy, brain edema, chronic ache, and tardive dyskinesia.

The above-mentioned reports are seen, for example, in "Endogenous factors protecting neurons against glutamate cytotoxicity" of "Course of Medical Science", Vol. 186, No. 11, Sept. 12, 1998, pages 787-790 (1998); "Control of Neuronal Death by Endogenous Factors" of "Folia Pharmacol. Jpn., 112, 177-186 (1998); and "Glutamate in CNS Disorders" of DN&P 8(5), 261-277, June, 1995.

Japanese Patent Publication No. 8-26069 describes that a peptide of the formula of pGlu-Asn-Ser-Pro-Arg-Gly-NH₂ and a pharmacologically acceptable salt thereof are of value as antidementia agents for improving cognition and dysmnesia of dementia patient. However, there are no reports on inhibition of glutamate toxicity of the above-mentioned peptide, its analogous peptide named Arginine vasopressin [AVP(1-9)], and its active metabolite in brain, called [pGlu⁴, Cyt⁵] AVP (4-9) .

### [DISCLOSURE OF THE INVENTION]

The invention has an object to provide a novel agent for use for treating nerval diseases and a novel inhibitor of glutamate toxicity, exclusive of an antidementia agent.

The present inventors have made study on pharmacological actions of the peptide of the formula of pGlu-Asn-Ser-Pro-Arg-Gly-NH₂ and a pharmacologically acceptable salt thereof, and discovered that this peptide and the salt show excellent inhibitory action on glutamate toxicity (protection on glutamate-induced neurocyte disorder). The invention has been completed on this discovery.

Accordingly, the present invention resides in an agent for use for treating nerval diseases which contains as an active component a peptide of the formula of:
pGlu-Asn-Ser-Pro-Arg-Gly-NH₂
or a pharmacologically acceptable salt thereof.

Each of the abbreviations in the formula of the peptide of the invention stands for the specific amino acid. As is well known, each of the abbreviations represents the following amino acids:
- pGlu:: pyroglutamic acid
- Asn:: asparagine
- Ser:: serine
- Pro:: proline
- Arg:: arginine
- Gly:: glycine

The optical configuration of each amino acid is L-form, unless otherwise indicated.

### [PREFERRED EMBODIMENTS OF THE INVENTION]

The above-mentioned peptide and its pharmacologically acceptable salt, which is the active component of the agent for treating nerval diseases or the inhibitor of glutamate toxicity according to the invention can be obtained by the methods described in the aforementioned Japanese Patent Publication No. 8-26069. Examples of the pharmacologically acceptable salts include acetate, hydrochloride, citrate, and methanesulfonate. Preferred is acetate.

The below-mentioned pharmacological experiments show that a pharmaceutical agent containing the aforementioned peptide or its pharmacological acceptable salt is of value as an inhibitor acting on glutamate toxicity.

According to the in vitro pharmacological experiments (using cells of rat cerebral cortex) for measuring actions on glutamate-induced neurocyte disorder, pGlu-Asn-Ser-Pro-Arg-Gly-NH₂ acetate showed an inhibitory action against glutamate-induced cellular death at a concentration of the range of 1/1,000 to 1/10,000, in comparison with AVP(4-9) which is a vasopressin metabolite. See the below-described Example 1. Accordingly, it has been confirmed that the pGlu-Asn-Ser-Pro-Arg-Gly-NH₂ acetate shows a inhibitory action on excessive exitatory actions and neuropathic actions induced by an agonist of glutamate acceptor.

On the basis of the above-mentioned results, that is, the peptide of the formula of pGlu-Asn-Ser-Pro-Arg-Gly-NH₂ and a pharmacologically acceptable salt thereof have an inhibitory action on the glutamate toxicity, it is expected that these compounds can be utilized as the agents for treating various nerval diseases including amyotrophy, scleroma of lateral funiculus of spinal, injury of eyes, retinopathy, It is furthermore expected that these compounds can be utilized for treating muscle spasm, convulsion, migraine, incontinence of urine, breakaway from nicotine, chronic ache, and tardive dyskinesia.

The above-mentioned peptide or its pharmacologically acceptable salt, which is an active component of the agent of the invention for treating nerval diseases can be generally administered parenterally (for instance, by intravenous injection, subcutaneous injection, intraventricular administration, syringomyelic administration, nasal administration, or rectal administration). If appropriate, the compounds can be orally administered. The pharmaceutical agent can be prepared in the form of injection liquid, nasal drop, suppository, percutaneous administrable agent, pellets, capsules, powder, or granules. If the parenterally administrable is an injection liquid, the injection liquid can be prepared using distilled water for injection liquid, physiological brine, or Ringer's solution. The injection liquid can be prepared in the form of freeze-dried ampul agent using an additive such as mannitol or sorbitol. The freeze-dried ample agent can be dissolved when it is administered.

The aforementioned peptide and its pharmacologically acceptable salt can be administered generally at a dose of 0.1 ng to 1 mg per day for adult. However, in the case of parenteral administration or nasogastric administration, the dose preferably is in the range of 0.1 ng to 100 µg. In the case of oral administration and rectal administration, the dose preferably is 10 to 1,000 times as much as the dose for parenteral administration. The dose can be adjusted in consideration of age, race, and conditions of patient.

The present invention is further described by the following examples.

### [Example 1] Action on glutamate-induced neurocyte disorder (in vitro)

### [Procedures]

The cerebral cortex of rat (0-day-old) was incubated at 37°C in a Hank's solution containing 1.5 mg/mL of dispase, and then cells were isolated. The neurocytes were dispersed on a poly-L-lysine-coated multiple well plate at a density of approx. 4 x 10⁴ cells/cm², and then to the plate was added cytosine-β-arabinofuranoside (20 µM). The nerocytes were cultured on the plate for 4 days.

The nerocytes were identified by indirect fluorescent method using a neuro-filament antibody

The cultured neurocytes were washed with a Hank's solution and further incubated at 37°C for 30 minutes under atmospheric conditions. Incubated neurocytes were again incubated for 10 minutes in a Hank's solution containing glutamate. Then, they were washed, and further incubated for one hour. At 15 minutes before the incubation was complete, propidium iodide (4.6 µg/mL) was added. After the incubations were complete, the incubated neurocytes were washed with PBS (phosphate buffer solution containing sodium chloride), and then observed using fluorescence microscope.

The pGlu-Asn-Ser-Pro-Arg-Gly-NH₂ acetate or AVP(4-9) was added to the 4 day-cultured solution prior to the glutamate treatment.

### [Results]

The pGlu-Asn-Ser-Pro-Arg-Gly-NH₂ acetate (4 day treatment) showed an inhibitory action against neuronal death induced by glutamate (1 mM) at either concentration of 0.1 nM and 1 nM. Further, it was noted that the inhibitory action of pGlu-Asn-Ser-Pro-Arg-Gly-NH₂ acetate against glutamate toxicity was reduced by the action of [Pmp¹, Tyr(Me)²]AVP (either of 0.1 nM and 1 nM) which was known as V1 receptor antagonist. In contrast, AVP(4-9), namely, vasopressin metabolite, inhibited the glutamate-induced neuronal death at either concentration of 1 µM and 10 µM.

The experimental results indicate that pGlu-Asn-Ser-Pro-Arg-Gly-NH₂ acetate shows an inhibitory-action against glutamate-induced cellular death at a concentration of 1/1,000 to 1/10,000, in comparison with AVP(4-9).

### [Example 2] Preparation example for injection solution

In 100 mL of distilled water for injection were dissolved 1 mg of pGlu-Asn-Ser-Pro-Arg-Gly-NH₂ acetate, 750 mg of sodium chloride, 150 mg of citric acid, and 370 mg of sodium citrate. The resulting solution was filtered, sterilized, and placed in a 1 mL-volume ampul. The ampul was sealed by fusing at its head, to give an injection solution according to the invention for treating nerval diseases.

### [Example 3] Preparation example for freeze-dried injection solution

In 100 mL of distilled water for injection were dissolved 1 mg of pGlu-Asn-Ser-Pro-Arg-Gly-NH₂ acetate, 2.5 g of D-mannitol, and 500 mg of sodium chloride. The resulting solution was filtered, sterilized, and placed in vials in an amount of 1 mL each. The solutions of the vials were freeze-dried to give a freeze-dried injection solution according to the invention for treating nerval diseases.

### [Example 4] Toxicity test

pGlu-Asn-Ser-Pro-Arg-Gly-NH₂ acetate was subcutaneously injected to a SD strain rat at a dose of 1 mg/kg. No noticeable changes were observed.

### [Possible Industrial Utilization]

The peptide of the formula of pGlu-Asn-Ser-Pro-Arg-Gly-NH₂ and a pharmacologically acceptable salt according to the invention show no specific toxicity in the conventional toxicity test, while they show excellent action for treating nerval diseases.

## Claims

1. Use of a peptide having the formula of:
pGlu-Asn-Ser-Pro-Arg-Gly-NH₂
or a pharmacologically acceptable salt thereof for the preparation of a medicament for the treatment of nerval diseases selected from the group consisting of amyotrophy, scleroma of lateral funiculus of spinal, injury of eyes, retinopathy, muscle spasm, convulsion, migraine, incontinence of urine, breakaway from nicotine, chronic ache, and tardive dyskinesia.

## Patentansprüche

1. Verwendung eines Peptids mit der Formel:
pGlu-Asn-Ser-Pro-Arg-Gly-NH₂
oder eines pharmakologisch annehmbaren Salzes davon zur Herstellung eines Medikaments für die Behandlung von Nervenerkrankungen, ausgewählt aus der Gruppe, bestehend aus Amyotrophie, Sklerom des lateralen Funiculus des Rückenmarks, Augenverletzung, Retinopathie, Muskelspasmus, Konvulsion, Migräne, Harninkontinenz, Abkommen bzw. Entzug von Nicotin, chronischem Schmerz und tardiver Dyskinesie.

## Revendications

1. Utilisation d'un peptide ayant la formule suivante:
pGlu-Asn-Ser-Pro-Arg-Gly-NH₂
ou d'un sel pharmaceutiquement acceptable de celui-ci pour la préparation d'un médicament pour le traitement des maladies nerveuses choisies dans le groupe consistant en l'amyotrophie, la sclérose du cordon latéral spinal, la lésion des yeux, la rétinopathie, le spasme musculaire, la convulsion, la migraine, l'incontinence urinaire, le sevrage de nicotine, la douleur chronique et la dyskinésie tardive.
